(19) Europäisches Patentamt
European Patent Office
Office européen des brevets

(11) **EP 3 801 434 B1**

(12) **EUROPEAN PATENT SPECIFICATION**

(45) Date of publication and mention
of the grant of the patent:
**09.02.2022 Bulletin 2022/06**

(21) Application number: **19725687.8**

(22) Date of filing: **17.05.2019**

(51) International Patent Classification (IPC):
***A61B 5/00*** *(2006.01)*      ***A61B 5/22*** *(2006.01)*
***A61H 1/02*** *(2006.01)*

(52) Cooperative Patent Classification (CPC):
**A61B 5/224; A61B 5/225; A61B 5/6806;**
**A61B 5/6825; A61B 5/6826; A61H 1/0237;**
**A61H 1/0281; A61H 1/0285; A61H 1/0288;**
A61H 2201/1215; A61H 2201/1238;
A61H 2201/1635; A61H 2201/165;
A61H 2201/5012; A61H 2201/5061;     (Cont.)

(86) International application number:
**PCT/EP2019/062790**

(87) International publication number:
**WO 2019/224117 (28.11.2019 Gazette 2019/48)**

(54) **METHOD AND SYSTEM FOR MEASURING ERGONOMIC LOAD**

VERFAHREN UND SYSTEM ZUR MESSUNG DER ERGONOMISCHEN BELASTUNG

MÉTHODE ET SYSTÈME DE MESURE DE CHARGE ERGONOMIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **25.05.2018 SE 1850631**

(43) Date of publication of application:
**14.04.2021 Bulletin 2021/15**

(73) Proprietor: **Bioservo Technologies Aktiebolag**
**164 40 Kista (SE)**

(72) Inventors:
• **EWALDSSON, Martin, Oskar, Gustaf**
**193 40 SIGTUNA (SE)**
• **SLEMAN, Jan, Johan, Fredrik**
**122 31 ENSKEDE (SE)**

(74) Representative: **Noréns Patentbyrå AB**
**Box 10198**
**100 55 Stockholm (SE)**

(56) References cited:
**EP-A1- 2 417 941**     **EP-A1- 2 762 123**
**WO-A1-2016/174083**     **US-A1- 2017 296 129**
**US-A1- 2017 344 919**

**(Cont. next page)**

(52) Cooperative Patent Classification (CPC): (Cont.)
A61H 2201/5069; A61H 2201/5071;
A61H 2201/5084; A61H 2201/5097

**Description**

The present invention relates to a method and a system for measuring ergonomic load and/or risk. In particular, it is related to such a method using a wearable, actively controlled force-assisting piece of equipment, such as an already existing such piece of equipment.

Conventionally, ergonomic load and risk is evaluated for various work tasks. For instance, evaluation models such as HAL-TLV (Hand Activity Level Threshold Limit Value) and KIM (Key Indicator Method) are used in industry, in which various movement patterns, postures, forces, as well as user fatigue and effort, are manually assessed and then used to calculate scores and similar values, in turn giving an indication of ergonomic risk for individual work tasks over time.

Such ergonomic risk measurement is very important, since the said scores can be used for early identification and prevention of ergonomic problems; for work force and production planning; and for development of production facilities. However, due to their manual character they are expensive and cumbersome to perform. In addition, the scoring is highly dependent on the measurement conditions, yielding high variation.

Also known is to use wearable, actively controlled, force-assisting equipment when performing various work tasks. For instance, such equipment in the form of different gloves is described in US 20130226350 A1 and WO 2008027002 A.

There are many types of such equipment, including not only gloves, but support gear for arms and/or legs, and even complete exoskeletons, for use in different situations. Common to all of these types of wearable equipment is that they use an active control loop, such as a feedback loop, to assist the wearing user to apply a particular force and/or to perform natural movement, which control loop is arranged to assist or amplify physical activities performed by a user using a force exerted by some type of motor. In many work situations, the user performing the task in question uses such a wearable piece of equipment.

As mentioned above, it would be desirable to be able to assess ergonomic risk in a better, more efficient, way. Also, it would be desirable to be able to plan, monitor and supervise ergonomically risky work tasks in a better way than what is the case today.

The present invention solves these and other problems, and in particular for work tasks that involve the use of a wearable actively controlled force assist equipment of the above described type.

Hence, the invention relates to a method when a user performs a work task, wherein the user wears and uses a wearable, actively controlled piece of force-assisting equipment used to assist the user in the performing of the said work task, which force-assisting equipment comprises a force-exerting means for assisting the user in applying a particular force and/or for performing movements of a particular type, and a sensor means for sensing a force applied by the user, wherein an assisting force exerted by said force-exerting means is feedback-controlled based on an instantaneous measurement value from the sensor means, which method is characterised in that the method comprises the steps of a) during the performance of said work task, measuring said measurement value using the sensor means for use in said feedback control; and b) using the measurement value to automatically calculate an actual ergonomic risk or load value for the performed work task using the said force-assisting equipment.

Moreover, the invention relates to a system for use when a user performs a work task, wherein the user wears and uses a wearable, actively controlled piece of force-assisting equipment used to assist the user in the performing of the said work task, which force-assisting equipment comprises a force-exerting means for assisting the user in applying a particular force and/or for performing movements of a particular type, and a sensor means for sensing a force applied by the user, wherein an assisting force exerted by said force-exerting means is feedback-controlled based on an instantaneous measurement value from the sensor means, which system is characterised in that the system comprises measuring means arranged to, during the performance of said work task, measure said measurement value using the sensor means for use in said feedback control, and in that the system furthermore comprising calculation means arranged to, using the measurement value, automatically calculate an actual ergonomic risk or load value for the performed work task using the said force-assisting equipment.

Furthermore, the invention relates to a computer software product for use when a user performs a work task, wherein the user wears and uses a wearable, actively controlled piece of force-assisting equipment used to assist the user in the performing of the said work task, which force-assisting equipment comprises a force-exerting means for assisting the user in applying a particular force and/or for performing movements of a particular type, and a sensor means for sensing a force applied by the user, wherein an assisting force exerted by said force-exerting means is feedback-controlled based on an instantaneous measurement value from the sensor means, which computer software product is characterised in that the computer software product is arranged to perform the steps of a) during the performance of said work task, performing said feedback control loop; b) also during the performance of said work task, receiving said measurement value from the sensor means for use in said feedback control; and c) using the measurement value to automatically calculate an actual ergonomic risk or load value for the performed work task using the said force-assisting equipment.

In the following, the invention will be described in detail, with reference to exemplifying embodiments of the invention and to the enclosed drawings, wherein:

> Figures 1 and 2 are respective schematic overviews, as seen from two different angles, of a system according to the present invention using a force-assisting glove 110, arranged to perform a method according to the present invention; and
> Figure 3 is a flowchart illustrating a method according to the present invention.

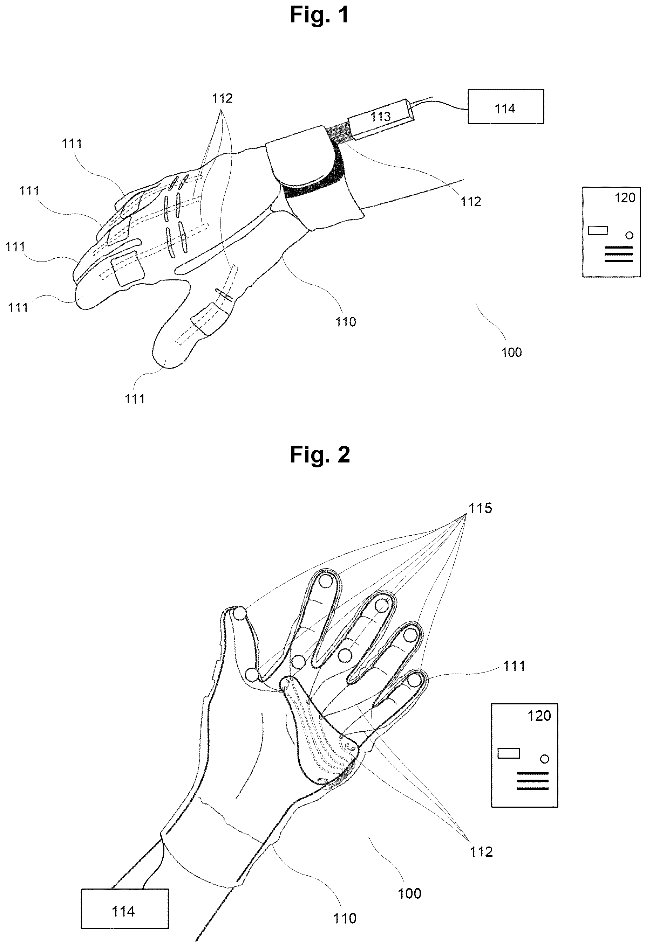

Hence, Figures 1 and 2 show a system 100 according to the present invention, for use when a user performs a particular work task. The work task may be any industrially performed work task involving the user in question manually performing work and/or applying a force.

Examples include the mounting of a screw; the lifting or carrying of an article, the operation of a piece of machinery, and so forth.

In general, during the course of the performance of such a work task, the user wears and uses a wearable, actively controlled piece of force-assisting equipment 110 used to assist the user in the performing of the said work task.

Examples of such force-assisting equipment 110 include various types of exoskeleton-type and similar wearable tools, aiding the user in performing particular activities involving the application of force and/or performing movements. Such forces and/or movements may, for instance, involve only one joint of the user, a particular body part or even substantially the whole muscular controlled body of the user, as the case may be. One particular example is a wearable strengthening glove, such as a glove using artificial tendons running across the fabric of the glove for assisting the user's applied hand and finger force. Another particular example is a similarly wearable shoulder assisting device, using artificial tendons to aid a user raising his or her arm at the shoulder joint.

When the user performs a particular work task using a particular force-assisting equipment having certain force sensors, the methodology of the present invention can be applied to the force-assisting equipment as a whole, taking into consideration measurement values from all force sensors that are relevant to the work task in question performed. It is also possible to use the present invention to evaluate the ergonomic risk or load of only a part of the assisted body part of the user, taken in isolation. For instance, using a force-assisting glove arranged to assist the user in applying force in each of the hand's five fingers, only one or some of the fingers can be continuously evaluated with respect to actual and/or hypothetical risk or load based upon measured forces from sensors only with respect to that or those fingers in question.

That the force-assisting equipment 110 is "actively controlled" means that its operation is arranged for being under active control based upon dynamically changing conditions, such as changing sensed forces, movements, accelerations, angles, postured, and so on. This active control is performed using an active control loop. One particularly interesting example of such an active control loop is a feedback control loop controlling the assisting force instantaneously applied by the equipment 110 in question, taking one or several of the below discussed sensor measurement data as input to the feedback loop.

In Figures 1 and 2, as an example of a force-assisting equipment 110 a strengthening glove is shown, comprising glove fingers 111 with artificial tendons 112 driven by an electric or hydraulic motor unit 113 under the control of an electronic control unit 114.

Moreover according to the present invention, the force-assisting equipment 110 in question comprises a force-exerting means for assisting the user in applying a particular force and/or for performing movements of a particular type, as a part of performing the said work task. Such a force-exerting means may, for instance, be the motor 113 arranged to apply a particular force to the artificial tendons 112 in turn arranged to pull at a particular point on the user's body so as to assist the user to apply a force and/or to perform a movement.

Also, the force-assisting equipment 110 comprises a sensor means 115 for sensing an instantaneous force applied by the ergonomically aided user during the performance of the work task in question. Preferably, the sensor means 115 may be arranged to measure such an instantaneous force continuously or repeatedly at least several times per second. The sensor means 115 may be any suitable force sensor unit, and may be connected to the control unit 114.

The sensor means 115 may be arranged to measure a pressing force between a body part of the user and a particular object currently being used or manipulated by the user during the course of the work task performed, such as a pressing force between the inside of the user's finger and a gripped object; or a rotational force applied about a joint of the user's body. The sensor means 115 may furthermore be arranged to measure additional aspects, such as angles or postures of the various body parts of the user.

In some embodiments, the sensor means 115 may also be an indirectly sensing arrangement. For instance, the sensor means may comprise a posture or angle sensor, such as arranged to measure a current angle of extension of an arm of the user in relation to the vertical. Then, using known information about the specific biomechanical system in which the force-assisting equipment is used, such as how the gravitational pull of the arm varies with the measured arm angle, the sensor means can calculate a force value which may then be used by the present invention.

Then, the force-assisting equipment 110 is arranged to control an assisting force exerted by the force-exerting means in the way described above. Such control of the assisting force is preferably performed in real-time, as the user performs the work task in question. For instance, the magnitude of the assisting force may be updated continuously, or at least several times per second, during the performance of the work task. For instance, the force-assisting equipment 110 may be arranged to apply an aiding force which is proportional to a corresponding force applied by the skeletal muscles of the user; or to regulate a predetermined constant force between the user's body and a manipulated object.

According to the invention, the control of the assisting force is a feedback control, performed based on an instantaneous force measurement value from the sensor means 115. For instance, the feedback control may be arranged to, during a particular work task or a particular moment or subpart of a work task, allow the user to apply a predetermined force or to allow the user to apply an artificially amplified force based on a measured force instantaneously applied by the user's own skeletal muscles. In the particular examples mentioned above, the assisting force may be a hand gripping force and an arm lifting force, respectively.

Figures 1 and 2 also disclose a computer or server 120, which may be comprised in or connected to the force-assisting equipment 110. The computer or server 120 may be a standalone piece of hardware, a distributed computer functionality, a remotely accessible computer functionality, and so on. In general, the feedback control described above may be performed by a control unit 114 which is actually a part of the force-assisting equipment 110 hardware, physically arranged on or in close proximity to the user's body. However, part or the whole of the feedback control may be performed by the computer or server 120 instead of, or in cooperation with, the control unit 114, as the case may be. In other cases, the functionality performed by the computer or server 120 may be entirely encompassed by the control unit 114.

It is realized that the system 100 according to the present invention preferably comprises both the force-assisting equipment 110 and the computer or server 120. However, the inventive system 100 may also be post-installed on or using an already-existing force-assisting equipment. Such post-installation may be performed by connecting the force-assisting equipment, wirelessly or using a wire, to the computer or server 120 in turn running computer software arranged to perform the necessary steps of a method according to the present invention. Alternatively, such software may be installed and executed on an existing control unit 114 and/or an existing computer or server 120 to which the force-assisting equipment 110 is already connected. Hence, the system 100 may comprise both the force-assisting equipment 110 and the computer or server 120 including any software arranged to, when executed, perform the method steps included in a method according to the invention. As an alternative, the invention may be implemented as such a software function, which in turn may be installed for execution on or from the existing force-assisting equipment 110 and/or the existing computer or server 120.

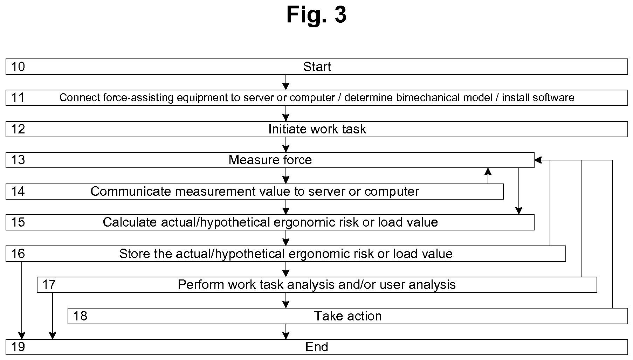

Figure 3 illustrates a method according to the present invention. In a first step 10, the method starts.

In an optional subsequent step 11, the force-assisting equipment 110 is connected to the server or computer 120, alternatively the computer or server 120 is arranged as a part of the force-assisting equipment 110, as described above, for automatic communication of said force measurement data value from said fore-assisting equipment 110 to said server or computer 120. Also in step 11, the said software may be installed, such as on the existing hardware.

In a subsequent step 12, the work task is initiated. As described above, the work task may be any work task during which the work task performing user uses the force-assisting equipment 110 for better performing the work task in question.

In a subsequent step 13, performed during the performance of said work task, the abovediscussed force measurement value is measured using the sensor means 115. As also described above, the force measurement value is measured for, and used in, said feedback control performed by the control unit 114 to aid the user in the work task in question the performance of which is ongoing. Such force measurement may hence be performed using the sensors 115 and for instance be performed continuously or intermittently. In general, the force measurement value will be representative of a force presently applied by the user, by aid of the force-assisting device 110, onto an object with which the user currently is working during the course of the said work task. It is understood that the force measurement value may pertain to one or several individual forces. For instance, each of the force sensors 115 may be individually sampled continuously or intermittently. In step 13, a movement and/or posture can also be measured, as described below.

In case the server or computer 120 was connected in step 11, in a subsequent step 14, said force measurement data value or values is or are communicated from the force-assisting equipment 110 to the server or computer 120.

In a subsequent step 15, the said measurement data is used, by the control unit 114, the computer or server 120 and/or the computer software, as discussed above, to automatically calculate an actual, current, ergonomic risk or load value for the performed work task in question, which work task is performed using the same force-assisting equipment 110 which is used to assist the user physically when performing said work task.

It is particularly preferred that the force-assisting equipment 110 is an existing force-assisting equipment which is not modified specifically for being used in the present method. In this case, step 15 may be performed by the said computer software executed on or from said server or computer 120.

In a subsequent step 16, the calculated actual ergonomic risk or load value is stored, in a database or memory accessible by the said computer software, such as in the control unit 114 and/or in the server or computer 120.

Thereafter, in a step 19, the method ends. However, before step 19, the method may reiterate, from step 16, any number of times back to step 13, such as for the duration of at least part or, or even the whole of, the work task in question. Hence, steps 13-15 or 13-16 may be performed repeatedly or continuously during the performance of at least part of the work task, or even during the whole performance of the work task in question.

Is illustrated in Figure 3, the method may also reiterate back to step 13 from step 14, in which case several force measurement values may first be recorded, and the actual ergonomic risk or load may be calculated based upon such a batch of force measurement values.

As mentioned, the force value is measured using the same sensors 115 that are used in the force-assisting feedback loop used to assist the user in the work task. Since the system 100 has up-to-date knowledge of the assisting force being applied, such as via tendons 112, the system 100 can calculate the force actually currently imparted by the skeletal muscles of the user, for instance as a simple subtraction from a measured contact force of the applied assisting force transformed to a corresponding applied contact force at the sensor 115 in question.

Since the system 100 also has knowledge about which sensor 115 provides which force measurement value, it is possible to relatively easily achieve a detailed view of the forces currently being applied by the user's skeletal muscles, and hence the ergonomic situation of the user at each respective moment in time. This may even be achievable without a specific calibration step.

In order to achieve such a detailed view of the currently imparted forces of the user, it is preferred that at least 3, preferably at least 5, force sensors are used simultaneously by the system 100 for performing such force measurements continuously or intermittently. Furthermore, it is preferred that a respective assisting force is applied using at least three individually and simultaneously operating force assisting means, such as the tendons 112 that are individually pulled by the motor 113. In order to calculate a current ergonomic status of the user, the force measurement values and/or the assisting forces may be transformed into a common geometry in which they can be compared directly one to the other. Such transforms may be part of the physical ergonomic model discussed below.

It is understood that, in the system aspect of the present invention, the system 100 comprises force measuring means arranged to, during the performance of said work task, perform the said force measurement using the sensor means 115 for use in said feedback control. Hence, the measuring means may, for instance, comprise the sensors 115 and the control means 114 arranged to receive the read sensor data from the sensors 115. Moreover, in this case the system 100 furthermore comprises calculation means, such as the above discussed computer software, which calculation means is arranged to, using the said force measurement data, automatically calculate said actual ergonomic risk or load value for the performed work task using the force-assisting equipment 110. In general, the system 100 may comprise the software described herein as a subpart.

Using such a method and such a system 100, it is hence possible to achieve an accurate, up-to-date and real-time estimation of an actual ergonomic risk or load for the user actually and currently performing the work task in question. Such ergonomic risk or load value may be used in a variety of different ways, as will be further detailed below and in the following.

For instance, when applied to a group of users performing one and the same work task, users having a relatively high actual ergonomic risk or load can be automatically identified, indicating potential maluse of a tool or similar; or users not being properly trained for that work task may be automatically identified.

The calculated actual ergonomic risk or load can also be monitored over time and used as an input for automated work task rotation. Preferably, the said computer software is connected to a work planning database, and comprises algorithms for automatically adapting a work task rotation plan as a result of an unexpectedly high calculated actual ergonomic risk or load value for at least one user. For instance, the value may be regarded as unexpectedly high if the corresponding actual ergonomic risk or load value for the user in question and for the same or corresponding work task jumps more than a predetermined amount or percentage over a particular maximum or predetermined time interval. In this case, the user in question may be assigned to a less ergonomically straining work task so as to avoid overwork for that particular user. In other embodiments, a work task rotation plan can be completely automatically proposed by the said computer software based upon recently calculated actual ergonomic risk or load values, for instance by minimizing a risk for overwork for individual identified users.

The calculated actual ergonomic risk or load values may also be used to evaluate and iteratively adapt changes in a production process involving manual work by individual users, such as the introduction of new work flows or tools. By quickly identifying possibly risky work tasks, the production process may be automatically and preferably immediately adjusted so as to avoid overworked users. The said computer software is preferably arranged to continuously monitor the calculated actual ergonomic risk or load values and set off an alert in case a particular work task is identified as producing higher actual ergonomic risk or load values than a predetermined threshold value, either on average across several users or for more than a predetermined number of individual users.

Hence, the present invention can be used as a way to improve the long-term and short-term health of users in a particular production flow, and generally results in lower costs for healthcare.

It is understood that, in order to be able to perform the above tasks, the computer software preferably has access to, such as via an updated database of the above mentioned type, with information regarding what work task each user is currently performing, and what individual user is currently using the force-assisting equipment 110.

Additional benefits of the present invention relate to production process improvements. For instance, particularly low-risk or low-load work tasks can be automatically identified by the computer software, and this information can be used by the computer software to automatically propose or implement an increase of production speed for such identified work tasks. The opposite is true regarding particularly high-risk or high-load work tasks, the speed of which may be automatically decreased. By mapping a set of work tasks in a particular production flow, ergonomic risks can be identified and handled by automatically adapting the production planning. For instance, identified particularly high-risk or high-load work tasks can be distributed to a larger subset of the working users, so as to avoid overworked users.

Furthermore, the invention can be used during education of new users. For instance, a user under education can perform a particular work task and automatically and preferably immediately receive feedback regarding the calculated actual ergonomic risk or load value resulting from the work. Then, the user may implement iterative adjustments in his or her technique so as to improve the ergonomics while performing the work task.

Moreover, the production quality may be improved using the present invention by reducing user malpractice due to fatigue. This is achieved not only as a consequence of avoiding overworked users, but also by decreasing fatigue from an acceptable level to an even lower level. All this can be performed automatically, and without adding any manual steps.

It is noted that the said computer software may preferably be arranged with algorithms for automatically performing these method steps, and to either produce a suggestion for planning adjustments to an operator, or automatically implement such adjustments in a production planning which is automatically supplied by the computer software.

The present invention can also be used for workplace certification purposes; for decreasing the need for subjective judgements regarding ergonomics; and for general workplace ergonomics mapping purposes.

It is noted that the computer software, using the actual ergonomic risk or load values calculated for a set of work tasks and for a set of users, for all the scenarios described above, can be used to assess the ergonomic status of both particular work tasks (independently of user) and for particular identified users (independently of work task). Then, combinations between these two estimations may be used for additional purposes. For instance, the ergonomic strain for a particular user performing a particular work task can be automatically assessed based upon a "normal" ergonomic strain for an average or typical user performing the work task in question.

Analyses and actions of the above discussed types are performed in an optional step 17. Then, the method may also reiterate back to step 13 from step 17, as the case may be.

In some embodiments, in addition to or instead of the said actual ergonomic risk or load value, in step 15 a hypothetical ergonomic risk or load value is also calculated for the performed task. This hypothetical ergonomic risk or load value is calculated based upon the same force measurement value as the corresponding actual ergonomic risk or load, and so as to reflect the hypothetical situation in which the work task in question was performed without the use of the force-assisting equipment 110. In other words- if the user performed the work task without using the force-assisting equipment 110, what would the ergonomic risk or load be then?

Thus, the hypothetical ergonomic risk or load value is calculated based upon to what extent the user is assisted by the force-assisting equipment 110, in turn determined by the currently measured force using which the force-assisting equipment 110 aids the user in performing the work task in question. For instance, in a simple exemplifying case the hypothetical ergonomic risk or load value may be calculated based upon the actual measured force applied by the user to the workpiece, as measured by the sensors 115, irrespective of any aiding force applied by the force-assisting equipment 110 with the purpose of aiding the user in performing the work task in question.

Then, the calculated actual ergonomic risk or load value is stored, in step 16, possibly together with the calculated hypothetical ergonomic risk or load value as described above.

The hypothetical ergonomic risk or load value may be used in an automatic production process planning algorithm performed by the said computer software, in which it is assessed what work tasks require the use of force-assisting equipment 110; what categories of users should be assigned to what work tasks; what work task rotation schemes to apply; and so on.

In some embodiments, the instantaneous hypothetical ergonomic risk or load value, or the hypothetical ergonomic risk or load value measured over time, may be used in combination with the corresponding actual ergonomic risk or load value measured instantaneously or over time, in order to further improve the quality of a production process. For instance, by considering the instantaneous or time-integrated hypothetical ergonomic risk, harmful work tasks can be identified, such as by allowing workers to perform a number of potentially harmful tasks while performing ergonomic risk measurements as described herein. For each identified such harmful work task, the instantaneous or time-integrated actual ergonomic risk, as measured from the same test runs, can be used to determine whether or not the use of force-assisting equipment is enough to reach ergonomic goals, or if the work task needs to be redesigned so as to be less ergonomically challenging.

Measured hypothetical and/or actual ergonomic risks or loads may also be used in a feedback loop for control of the force-assisting equipment itself. For instance, a currently measured instantaneous and/or time-integrated actual ergonomic risk or load value can be used as an input value or trigger for a modification of a control program used by the force-assisting equipment used to perform the same work task in relation to which the said ergonomic risk or load value is currently being measured. Such feedback is hence performed in realtime or near realtime. For instance, if an actual ergonomic risk which is higher than an allowed threshold value is detected, the control program can be adjusted so as to provide stronger force-assisting action by the force-assisting equipment. For instance, the force-assisting equipment may, in its own force-assisting feedback loop program, use a parameter providing a certain force-assist as a percentage of a corresponding force currently applied by the user, in which case this parameter is modified upwards as a result of the detected unacceptably high ergonomic risk. Another example is when the said control program implements a maximum force-assist value, which maximum force-assist value may be increased as a result of the detected unacceptably high ergonomic risk.

As understood from the above, the measurement step 13 may be performed instantaneously, at a particular point in time. Furthermore, the calculation step 15 may be performed based on a plurality of different such instantaneous measurement values from a certain time period during the performance of the work task.

According to a very preferred embodiment, the method further comprises an initial step, such as a part of step 11, in which a physical ergonomic model, which is specific to the particular work task or to the particular piece of force-assisting equipment 110, or preferably specific to both these aspects, is determined. The physical ergonomic model is further determined based upon physical characteristics of the work task in question, as well as physical characteristics of the sensor means 115, respectively, as the case may be. It is important that the physical ergonomic model is fully determined before step 15, in the sense that the calculation(s) performed in step 15 can be fully automated and not require any manual input from an operator. In particular, it is preferred that the physical ergonomic model determined in step 11 is used as a basis for the calculation(s) in step 15.

According to the physical ergonomic model, the said force measurement data is used as an input to the physical ergonomic model, and the actual and/or hypothetical ergonomic risk or load value is calculated as an output based on the said input force measurement data. Hence, the above mentioned geometric transforms may be a part of the physical ergonomic model. As an additional example, the physical ergonomic model may take into numerical consideration the fact that certain types of movements may be associated with different types of relationships between applied force, movement and ergonomic risk or load.

In general, the actual and/or hypothetical ergonomic risk or load may be calculated in step 15 based upon the measured force data from the sensors 115. However, it is understood that the sensors 115 may also comprise movement and/or posture sensors, the measurement values of which may also be input, such as via the control unit 114, to the physical ergonomic model and used to calculate the actual and/or hypothetical ergonomic risk or load. Hence, the currently applied force may result in different ergonomic risks or loads depending on a motion pattern currently being pursued by the user and/or a current posture of the relevant body part of the user.

In a particular embodiment, the said physical ergonomic model comprises an implementation of a HAL-TLV (Hand Activity Level Threshold Limit Value) and/or KIM (Key Indicator Method) evaluation model, where the input data of the respective evaluation model are completely provided in an automatic way based upon the read sensor 115 data as described above. These evaluation models have been proven to yield relevant values, but have not to date been useful in fully automated applications.

In an exemplifying example, incorporated herein in order to improve the understanding of how to implement the present invention in practise, it was assessed how much ergonomic risk a particular person was subjected to when doing three different grasp intensive work tasks while using a force-assisting equipment in the form of a force-assisting glove. Both the hypothetical risk level and the actual risk level were assessed for these work tasks. A HAL-TLV was used as ergonomic evaluation model and tailored to the particular force-assisting glove used. The HAL-TLV model used has two risk levels that are special, an action level (AL) threshold value = 0.56 and a threshold limit value (TLV) = 0.78. When a risk level calculated based upon measurements is below the AL value, the ergonomic risk is considered low, and no action is required. When the calculated risk is between AL and TLV, a redesign of the work place should be considered. If the risk is above TLV, a redesign of the work place is considered necessary.

When the ergonomic risk level had been evaluated, a particular action based on the HAL-TLV ergonomic evaluation model was proposed. In this example, this was made manually. However, it is realized that such proposing can be made automatically as described herein.

The actual risk when using the force-assisting equipment is lower than the hypothetical risk level, since the force-assisting equipment applies force when the user grasps objects, thereby reducing the force the user needs to apply to do the work task in question.

As mentioned above, three different tests were performed according to the following.

- A paving stone weighing 3 kg was lifted a short distance and then put back down. This test simulates a work task where paving stones are placed next to each other.

- Buttons were pinched together in quick succession using the user's thumb and the index finger. This is to simulate pinching tasks.

- A screwdriver was used to fasten screws. It was actuated with the user's index finger as trigger. This simulates work tasks such as fastening screws ore bolts.

For each test, the grasping action was repeated 50 times and an average was calculated.

The ergonomic risk according to the HAL TLV evaluation model was calculated as follows:

$$HAL_{TLV\,SCORE} = \frac{FORCE}{FORCE_{MAX}} * \frac{10}{10 - \frac{6.56 ln(DUTYCYCLE)FREQUENCY^{1.31}}{1 + 3.18 FREQUENCY^{1.31}}},$$

Where $FORCE_{MAX}$ is a norm value which is determined based upon the user's maximum strength. Such calculations will be well-known for the person skilled in the relevant arts.

The following table summarizes the results of these three tests:

| Test | Hypothetical risk | Actual risk (with force-assisting equipment(FAE)) | Analysis | Action |
|---|---|---|---|---|
| Paving stone | 0.82<br>Force = 15.2N<br>Duty cycle = 40%<br>Frequency=0.25Hz | 0.36<br>Force = 6.6N<br>Duty cycle = 40%<br>Frequency=0.25Hz | 0.82 > TLV → High risk.<br><br>0.36 < AL → Low risk<br>This work task should not be performed without the FAE | Use the FAE to reduce the risk level to an acceptable level |
| Button pinch | 0.91<br>Force = ION<br>Duty cycle = 36%<br>Frequency = 1.0Hz | 0.51<br>Force = 5.6N<br>Duty cycle = 36%<br>Frequency = 1.0Hz | 0.82 > TLV → High risk.<br><br>0.51 < AL → Low risk<br>This work task should not be performed without the FAE | Use the FAE to reduce the risk level to an acceptable level |
| Screwdriver | 0.59<br>Force = 12N<br>Duty cycle = 60%<br>Frequency = 0.15Hz | 0.28<br>Force = 5.7N<br>Duty cycle = 60%<br>Frequency = 0.15Hz | AL < 0.59 < TLV → Medium risk.<br>This work task can be performed without obvious risks but a lower risk is preferred | Be cautious. If the risk increases consider using an FAE. |

In a preferred embodiment, the force-assisting equipment is arranged specifically for assisting movements and/or force of the user's shoulder, arm, hand, leg and/or torso.

In a preferred embodiment, the method comprises as a part of step 17 an analysis and decision step, in which the calculated actual and/or hypothetical ergonomic risk or load value is used for taking a decision affecting how, if, for how long, or when the work task is performed.

In particular, the said decision step may comprise a comparison between firstly the calculated actual and/or hypothetical ergonomic risk or load value, in the form of an instantaneous ergonomic risk or load value or an integrated total ergonomic risk or load value, and secondly a predetermined corresponding threshold value. If the calculated actual and/or hypothetical ergonomic risk or load value is found to be higher than said threshold value, the method is arranged to achieve an alarm signal.

Herein, the term "alarm signal" should be determined broadly. Hence, apart from an audiovisual, physical alarm signal, the signal achieved may be a digital or electric signal signifying a particular state into which the system 100 enters as a result of the alarm signal. Hence, the system 100 may be put into a warning state or similar, such as with respect to the work task and the user in question for which the ergonomic situation was measured and evaluated. This state change of the system 100 may then, in turn, result in any of a number of possible outcomes, performed in an optional action taking step 18, performed after step 17.

For instance, in one exemplifying embodiment step 18 may comprise causing the force-assisting equipment 110 to limit the user's movements as a result of the detection of said alarm signal. Such limitation may be achieved in any suitable way, and may be of a forcing type or merely informing the user of the suitability of no longer performing the work task under the current conditions.

For instance, said limitation imparted in step 18 may be achieved by the force-assisting equipment 110 denying the user sufficient force assisting to be able to perform certain predetermined movements and/or movements requiring the user to use a force exceeding a force threshold value, or that the user is alerted regarding the detected alarm signal. This may be achieved by the said computer software, such as by the force-assisting equipment 110 itself and/or the computer or server 120. The information in question may be presented to the user via a suitable interface, such as a graphical display or warning lamp on the force-assisting equipment 110 and/or the computer or server 120.

Above, preferred embodiments have been described. However, it is apparent to the skilled person that many modifications can be made to the disclosed embodiments without departing from the basic idea of the invention.

For instance, as has been mentioned in a number of contexts above, the force-assisting equipment 110 may be arranged to assist the user in the imparting of a static and/or dynamic force, such as when using a screwdriver or pressing two objects together or apart. The force-assisting equipment 110 may in addition, or instead, be arranged to assist the user in the performing of various movements. All which has been said above relates equally well to both these cases, as applicable. In a more general sense, the force-assisting equipment 110 may be labelled as a "strengthening" equipment.

Furthermore, it is realized that the force-assisting equipment 110 may have any other properties in addition to the ones described above.

It is understood that all which has been said in relation to the present method is equally applicable to the present system 100, and vice versa.

Hence, the invention is not limited to the described embodiments, but can be varied within the scope of the enclosed claims.

**Claims**

1. Method when a user performs a work task, wherein the user wears and uses a wearable, actively controlled piece of force-assisting equipment (110) used to assist the user in the performing of the said work task, which force-assisting equipment (110) comprises a force-exerting means (112,113) for assisting the user in applying a particular force and/or for performing movements of a particular type, and a sensor means (115) for sensing a force applied by the user, wherein an assisting force exerted by said force-exerting means (112,113) is feedback-controlled based on an instantaneous measurement value from the sensor means (115), **characterised in that** the method comprises the steps of

   a) determine a physical ergonomic model, which is specific to said work task and/or to said piece of force-assisting equipment (110), based upon physical characteristics of the work task and/or physical characteristics of the sensor means (115), which model further comprises a transform from an assisting force applied by said force-assisting equipment to a corresponding force imparted by the user's skeletal muscles;
   b) during the performance of said work task, measuring said measurement value using the sensor means (115) for use in said feedback control; and
   c) using the measurement value and said physical ergonomic model to automatically calculate an actual ergonomic risk or load value for the performed work task using the said force-assisting equipment (110).

2. Method according to claim 1, **characterised in that** the method further comprises, before step a, connecting the force-assisting equipment (110) to a server or computer (120), alternatively arranging a server of computer (120) as a part of the force-assisting equipment (110), for automatic communication of said measurement value to said server or computer (120), and, before step b, communicating said measurement value from the force-assisting equipment (110) to the said server or computer (120).

3. Method according to claim 2, **characterised in that** the force-assisting equipment (110) is an existing force-assisting equipment which is not modified specifically for being used in the said method, and **in that** step b is performed by computer software executed on or from said server or computer (120).

4. Method according to any one of the preceding claims, **characterised in that**, in addition to the actual ergonomic risk or load value, a hypothetical ergonomic risk or load value is also calculated for the performed task reflecting the hypothetical case in which the said force-assisting equipment (110) is not used, which hypothetical ergonomic risk or load value is calculated based upon to what extent the user is assisted by the force-assisting equipment (110), and **in that** the calculated actual ergonomic risk or load value is stored together with the calculated hypothetical ergonomic risk or load value.

5. Method according to any one of the preceding claims, **characterised in that** steps a and b are performed repeatedly or continuously during the performance of at least part of the work task.

6. Method according to claim 5, **characterised in that** the calculation in step b is based on a plurality of different instantaneous measurement values from a certain time period during the work task.

7. Method according to any one of the preceding claims, **characterised in that** the actual ergonomic risk or load value according to the physical ergonomic model is calculated as an output based on the said measurement value.

8. Method according to claim 7, **characterised in that** the physical ergonomic model comprises a HAL and/or KIM evaluation model.

9. Method according to any one of the preceding claims, **characterised in that** the force-assisting equipment (110) is arranged specifically for assisting force of the user's shoulder, arm, hand, leg and/or torso.

10. Method according to any one of the preceding claims, **characterised in that** the method comprises a decision step in which the calculated actual ergonomic risk or load value, and possibly also a calculated hypothetical ergonomic risk or load value, is used for taking a decision affecting how, if, for how long, or when the work task is performed.

11. Method according to claim 10, **characterised in that** the decision step comprises a comparison between firstly the calculated actual ergonomic risk or load value, in the form of an instantaneous risk or load value or an integrated total risk or load value, and secondly a predetermined corresponding threshold value, and if the calculated actual ergonomic risk or load value is found to be higher than said threshold value to achieve an alarm signal.

12. Method according to claim 11, **characterised in that** the method comprises causing the force-assisting equipment (110) to limit the user's movements as a result of the detection of said alarm signal.

13. Method according to claim 12, **characterised in that** said limitation is achieved by the force-assisting equipment (110) denying the user sufficient force assisting to be able to perform a predetermined work task requiring the user to use a force exceeding a force threshold value, or that the user is alerted regarding the detected alarm signal.

14. System for use when a user performs a work task, wherein the user wears and uses a wearable, actively controlled piece of force-assisting equipment (110) used to assist the user in the performing of the said work task, which force-assisting equipment (110) comprises a force-exerting means (112,113) for assisting the user in applying a particular force and/or for performing movements of a particular type, and a sensor means (115) for sensing a force applied by the user, wherein an assisting force exerted by said force-exerting means (112,113) is feedback-controlled based on an instantaneous measurement value from the sensor means (115), **characterised in that** the system comprises measuring means arranged to, during the performance of said work task, measure said measurement value using the sensor means (115) for use in said feedback control, **in that** the system furthermore comprising calculation means arranged to, using the measurement value, automatically calculate an actual ergonomic risk or load value for the performed work task using the said force-assisting equipment (110), and **in that** the calculation means is further arranged to perform said automatic calculation using a determined physical ergonomic model, which model is specific to said work task and/or to said piece of force-assisting equipment (110), which model is determined based upon physical characteristics of the work task and/or physical characteristics of the sensor means (115), and which model further comprises a transform from an assisting force applied by said force-assisting equipment to a corresponding force imparted by the user's skeletal muscles.

15. Computer software product for use when a user performs a work task, wherein the user wears and uses a wearable, actively controlled piece of force-assisting equipment (110) used to assist the user in the performing of the said work task, which force-assisting equipment (110) comprises a force-exerting means (112,113) for assisting the user in applying a particular force and/or for performing movements of a particular type, and a sensor means (115) for sensing a force applied by the user, wherein an assisting force exerted by said force-exerting means (112,113) is feedback-controlled based on an instantaneous measurement value from the sensor means (115), **characterised in that** the computer software product is arranged to perform the steps of

a) during the performance of said work task, performing said feedback control loop;
b) also during the performance of said work task, receiving said measurement value from the sensor means (115) for use in said feedback control; and
c) using the measurement value to automatically calculate an actual ergonomic risk or load value for the performed work task using the said force-assisting equipment (110),

and **in that** the automatic calculation in step c is performed using a determined physical ergonomic model, which model is specific to said work task and/or to said piece of force-assisting equipment (110), which model is determined based upon physical characteristics of the work task and/or physical characteristics of the sensor means (115), and which model further comprises a transform from an assisting force applied by said force-assisting equipment to a corresponding force imparted by the user's skeletal muscles.

**Patentansprüche**

1. Verfahren, wenn ein Benutzer eine Arbeitsaufgabe ausführt, wobei der Benutzer ein tragbares, aktiv gesteuertes Teil einer Kraftunterstützungsausrüstung (110) trägt und verwendet, das verwendet wird, um den Benutzer bei der Ausführung der Arbeitsaufgabe zu unterstützen, wobei

die Kraftunterstützungsausrüstung (110) ein Kraftausübungsmittel (112, 113) zum Unterstützen des Benutzers beim Aufbringen einer bestimmten Kraft und/oder zum Ausführen von Bewegungen eines bestimmten Typs umfasst, und
eine Sensoreinrichtung (115) zum Erfassen einer vom Benutzer aufgebrachten Kraft, wobei eine durch die Kraftausübungseinrichtung (112, 113) ausgeübte Hilfskraft auf der Grundlage eines momentanen Messwerts von der Sensoreinrichtung (115) rückgekoppelt gesteuert wird,
**dadurch gekennzeichnet, dass**
das Verfahren die folgenden Schritte umfasst:

a) Bestimmen eines physikalischen ergonomischen Modells, das für die Arbeitsaufgabe und/oder die Kraftunterstützungsausrüstung (110) spezifisch ist, auf der Grundlage physikalischer Merkmale der Arbeitsaufgabe und/oder physikalischer Merkmale der Sensoreinrichtung (115), wobei
das Modell ferner eine Transformation von einer, durch die Kraftausübungseinrichtung aufgebrachten, Unterstützungskraft in eine entsprechende, von den Skelettmuskeln des Benutzers ausgeübte, Kraft umfasst;
b) Während der Ausführung der Arbeitsaufgabe den Messwert, unter Verwendung der Sensoreinrichtung (115), zur Verwendung in der Rückkopplungssteuerung, zu messen; und
c) Verwendung des Messwerts und des physikalisch-ergonomischen Modells zur automatischen Berechnung eines tatsächlichen ergonomischen Risiko- oder Belastungswerts für die durchgeführte Arbeitsaufgabe, unter Verwendung der Kraftunterstützungsausrüstung (110).

2. Verfahren nach Anspruch 1,
**dadurch gekennzeichnet, dass**
das Verfahren ferner umfasst, dass vor Schritt a die Kraftunterstützungsausrüstung (110) mit einem Server oder Computer (120) verbunden wird, alternativ ein Server oder Computer (120) als Teil der Kraftunterstützungsausrüstung (110) angeordnet wird, um den Messwert automatisch an den Server oder Computer (120) zu übermitteln, und dass vor Schritt b der Messwert von der Kraftunterstützungsausrüstung (110) an den Server oder Computer (120) übermittelt wird.

3. Verfahren nach Anspruch 2,
**dadurch gekennzeichnet, dass**
die Kraftunterstützungsausrüstung (110) eine bestehende Kraftunterstützungsausrüstung ist, die nicht speziell für die Verwendung in dem Verfahren modifiziert wird, und dass der Schritt b durch eine Computersoftware ausgeführt wird, die auf oder von dem Server oder Computer (120) ausgeführt wird.

4. Verfahren nach einem der vorhergehenden Ansprüche,

**dadurch gekennzeichnet, dass**
zusätzlich zu dem tatsächlichen ergonomischen Risiko- oder Belastungswert auch ein hypothetischer ergonomischer Risiko- oder Belastungswert für die ausgeführte Aufgabe berechnet wird, der den hypothetischen Fall widerspiegelt, in dem die Kraftunterstützungsausrüstung (110) nicht verwendet wird, wobei der hypothetische ergonomische Risiko- oder Belastungswert auf Grundlage dessen berechnet wird, in welchem Umfang der Benutzer durch die Kraftunterstützungsausrüstung (110) unterstützt wird, und dass der berechnete tatsächliche ergonomische Risiko- oder Belastungswert zusammen mit dem berechneten hypothetischen ergonomischen Risiko- oder Belastungswert gespeichert wird.

5. Verfahren nach einem der vorhergehenden Ansprüche,
   **dadurch gekennzeichnet, dass**
   die Schritte a und b wiederholt oder kontinuierlich während der Ausführung zumindest eines Teils der Arbeitsaufgabe durchgeführt werden.

6. Verfahren nach Anspruch 5,
   **dadurch gekennzeichnet, dass**
   die Berechnung in Schritt b auf einer Vielzahl von unterschiedlichen Momentanmesswerten aus einem bestimmten Zeitraum während der Arbeitsaufgabe basiert.

7. Verfahren nach einem der vorhergehenden Ansprüche,
   **dadurch gekennzeichnet, dass**
   der tatsächliche ergonomische Risiko- oder Belastungswert, gemäß dem physikalisch-ergonomischen Modell, als Ausgabe auf Grundlage des genannten Messwerts berechnet wird.

8. Verfahren nach Anspruch 7,
   **dadurch gekennzeichnet, dass**
   das physikalisch-ergonomische Modell ein HAL- und/oder KIM-Bewertungsmodell umfasst.

9. Verfahren nach einem der vorhergehenden Ansprüche,
   **dadurch gekennzeichnet, dass**
   die Kraftunterstützungsausrüstung (110) speziell zur Kraftunterstützung der Schulter, des Arms, der Hand, des Beins und/oder des Rumpfes des Benutzers angeordnet ist.

10. Verfahren nach einem der vorhergehenden Ansprüche,
    **dadurch gekennzeichnet, dass**
    das Verfahren einen Entscheidungsschritt umfasst, in dem der berechnete tatsächliche ergonomische Risiko- oder Belastungswert und möglicherweise auch ein berechneter hypothetischer ergonomischer Risiko- oder Belastungswert verwendet wird, um eine Entscheidung zu treffen, die beeinflusst, wie, ob, wie lange oder wann die Arbeitsaufgabe ausgeführt wird.

11. Verfahren nach Anspruch 10,
    **dadurch gekennzeichnet, dass**
    der Entscheidungsschritt einen Vergleich zwischen - erstens - dem berechneten tatsächlichen ergonomischen Risiko- oder Belastungswert, in Form eines momentanen Risiko- oder Belastungswerts oder eines integrierten Gesamtrisiko- oder Gesamtbelastungswerts, und - zweitens - einem vorbestimmten entsprechenden Schwellenwert umfasst, und dass, wenn festgestellt wird, dass der berechnete tatsächliche ergonomische Risiko- oder Belastungswert höher als der Schwellenwert ist, ein Alarmsignal erzeugt wird.

12. Verfahren nach Anspruch 11,
    **dadurch gekennzeichnet, dass**
    das Verfahren umfasst, dass die Kraftunterstützungsausrüstung (110) veranlasst wird, die Bewegungen des Benutzers als Folge der Erfassung des Alarmsignals zu begrenzen.

13. Verfahren nach Anspruch 12,
    **dadurch gekennzeichnet, dass**
    die Begrenzung dadurch erreicht wird, dass die Kraftunterstützungsausrüstung (110) dem Benutzer eine ausreichende Kraftunterstützung verweigert, um eine vorbestimmte Arbeitsaufgabe ausführen zu können, die es erfordert, dass der Benutzer eine Kraft einsetzt, die einen Kraftschwellenwert überschreitet, oder dass der Benutzer bezüglich des erkannten Alarmsignals alarmiert wird.

**14.** System zur Verwendung, wenn ein Benutzer eine Arbeitsaufgabe ausführt, wobei der Benutzer ein tragbares, aktiv gesteuertes Teil einer Kraftunterstützungsausrüstung (110) trägt und verwendet, das verwendet wird, um den Benutzer bei der Ausführung der Arbeitsaufgabe zu unterstützen, wobei

die Kraftunterstützungsausrüstung (110) eine Kraftausübungseinrichtung (112, 113) zum Unterstützen des Benutzers beim Aufbringen einer bestimmten Kraft und/oder zum Ausführen von Bewegungen eines bestimmten Typs und eine Sensoreinrichtung (115) zum Erfassen einer durch den Benutzer aufgebrachten Kraft umfasst, wobei eine, durch die Kraftausübungseinrichtung (112,113) ausgeübte, Unterstützungskraft, auf der Grundlage eines momentanen Messwerts von der Sensoreinrichtung (115) rückkopplungsgesteuert wird,
**dadurch gekennzeichnet, dass**
das System eine Messeinrichtung umfasst, die so eingerichtet ist, dass sie während der Ausführung der Arbeitsaufgabe den Messwert unter Verwendung der Sensoreinrichtung (115), zur Verwendung bei der Rückkopplungssteuerung, misst,
dass das System ferner eine Berechnungseinrichtung umfasst, die so eingerichtet ist, dass sie unter Verwendung des Messwerts automatisch einen tatsächlichen ergonomischen Risikooder Belastungswert für die ausgeführte Arbeitsaufgabe, unter Verwendung der kraftunterstützenden Ausrüstung (110), berechnet, und dass die Berechnungseinrichtung weiterhin angeordnet ist, um die automatische Berechnung, unter Verwendung eines bestimmten physikalisch-ergonomischen Modells, durchzuführen, das für die Arbeitsaufgabe und/oder die Kraftunterstützungsausrüstung (110) spezifisch ist, wobei das Modell auf der Grundlage physikalischer Merkmale der Arbeitsaufgabe und/oder physikalischen Eigenschaften der Sensoreinrichtung (115) basiert, und wobei das Modell ferner eine Transformation von einer, von der Kraftunterstützungsausrüstung aufgebrachten, Unterstützungskraft in eine entsprechende, von den Skelettmuskeln des Benutzers ausgeübte, Kraft umfasst.

**15.** Computersoftwareprodukt zur Verwendung, wenn ein Benutzer eine Arbeitsaufgabe ausführt, wobei

der Benutzer ein tragbares, aktiv gesteuertes Teil einer Kraftunterstützungsausrüstung (110) trägt und verwendet, das verwendet wird, um den Benutzer bei der Ausführung der Arbeitsaufgabe zu unterstützen, wobei
die Kraftunterstützungsausrüstung (110) eine Kraftausübungseinrichtung (112, 113) zum Unterstützen des Benutzers beim Aufbringen einer bestimmten Kraft und/oder zum Ausführen von Bewegungen eines bestimmten Typs, und
eine Sensoreinrichtung (115) zum Erfassen einer von dem Benutzer aufgebrachten Kraft umfasst, wobei eine von der Kraftausübungseinrichtung (112, 113) ausgeübte Hilfskraft auf der Grundlage eines momentanen Messwerts von der Sensoreinrichtung (115) rückkopplungsgesteuert wird,
**dadurch gekennzeichnet, dass**
das Computersoftwareprodukt so eingerichtet ist, dass es die folgenden Schritte ausführt:

a) Während der Ausführung der Arbeitsaufgabe die Rückkopplungsregelschleife durchführen;
b) Ebenfalls während der Ausführung der Arbeitsaufgabe, empfangen des Messwertes von der Sensoreinrichtung (115) zur Verwendung in der Rückkopplungssteuerung; und
c) Verwendung des Messwerts zur automatischen Berechnung eines tatsächlichen ergonomischen Risikooder Belastungswerts für die ausgeführte Arbeitsaufgabe unter Verwendung der Kraftunterstützungsausrüstung (110), und dass die automatische Berechnung in Schritt c unter Verwendung eines bestimmten physikalisch-ergonomischen Modells durchgeführt wird, wobei

das Modell für die Arbeitsaufgabe und/oder für das Kraftunterstützungsgerät (110) spezifisch ist, wobei das Modell auf der Grundlage physikalischer Merkmale der Arbeitsaufgabe und/oder physikalischer Merkmale der Sensoreinrichtung (115) bestimmt wird, und wobei das Modell ferner eine Transformation von einer, durch das Kraftunterstützungsgerät aufgebrachten, Unterstützungskraft in eine entsprechende, durch die Skelettmuskeln des Benutzers aufgebrachte, Kraft umfasst.

**Revendications**

**1.** Procédé lorsqu'un utilisateur réalise une tâche de travail, dans laquelle l'utilisateur porte et utilise une pièce pouvant être portée activement commandée d'équipement d'assistance de force (110) utilisée pour aider l'utilisateur à réaliser ladite tâche de travail, lequel équipement d'assistance de force (110) comprend un moyen d'exercice de force (112, 113) pour aider l'utilisateur à appliquer une force particulière et/ou pour réaliser des mouvement d'un type particulier, et un moyen de capteur (115) pour détecter une force appliquée par l'utilisateur, dans lequel une force d'assistance exercée par ledit moyen d'exercice de force (112, 113) est commandée par rétroaction sur la base d'une valeur de mesure instantanée du moyen de capteur (115), **caractérisé en ce que** le procédé comprend les étapes consistant à :

a) déterminer un modèle ergonomique physique qui est spécifique à ladite tâche de travail et/ou à ladite pièce d'équipement d'assistance de force (110), sur la base des caractéristiques physiques de la tâche de travail et/ou des caractéristiques physiques du moyen de capteur (115), lequel modèle comprend en outre une transformation d'une force d'assistance appliquée par ledit équipement d'assistance de force en une force correspondante communiquée par les muscles squelettiques de l'utilisateur ;

b) pendant la réalisation de ladite tâche de travail, mesurer ladite valeur de mesure à l'aide du moyen de capteur (115) destiné à être utilisé dans ladite commande de rétroaction ; et

c) utiliser la valeur de mesure et ledit modèle ergonomique physique pour calculer automatiquement une véritable valeur de risque ergonomique ou de charge pour la tâche de travail réalisée en utilisant ledit équipement d'assistance de force (110).

2. Procédé selon la revendication 1, **caractérisé en ce que** le procédé comprend en outre, avant l'étape a, l'étape consistant à raccorder l'équipement d'assistance de force (110) à un serveur ou à un ordinateur (120), agencer en variante un serveur d'ordinateur (120) sous la forme d'une partie de l'équipement d'assistance de force (110), pour la communication automatique de ladite valeur de mesure audit serveur ou ordinateur (120), et avant l'étape b, l'étape consistant à communiquer ladite valeur de mesure de l'équipement d'assistance de force (110) audit serveur ou ordinateur (120).

3. Procédé selon la revendication 2, **caractérisé en ce que** l'équipement d'assistance de force (110) est un équipement d'assistance de force existant qui n'est pas spécifiquement modifié pour être utilisé dans ledit procédé, et **en ce que** l'étape b est réalisée par logiciel, exécutée sur ou depuis ledit serveur ou ordinateur (120).

4. Procédé selon l'une quelconque des revendications précédentes, **caractérisé en ce que**, en plus de la véritable valeur de risque ergonomique ou de charge, une hypothétique valeur de risque ergonomique ou de charge est également calculée pour la tâche réalisée reflétant le cas hypothétique dans lequel ledit équipement d'assistance de force (110) n'est pas utilisé, ladite valeur hypothétique de risque ergonomique ou de charge est calculée en fonction de la mesure selon laquelle l'utilisateur est assisté par l'équipement d'assistance de force (110), et **en ce que** la véritable valeur de risque ergonomique ou de charge calculée est stockée conjointement avec l'hypothétique valeur de risque ergonomique ou de charge calculée.

5. Procédé selon l'une quelconque des revendications précédentes, **caractérisé en ce que** les étapes a et b sont réalisées à plusieurs reprises ou de manière continue pendant la réalisation d'au moins une partie de la tâche de travail.

6. Procédé selon la revendication 5, **caractérisé en ce que** le calcul à l'étape b est basé sur une pluralité de valeurs de mesure instantanées différentes provenant d'une certaine période de temps pendant la tâche de travail.

7. Procédé selon l'une quelconque des revendications précédentes, **caractérisé en ce que** la véritable valeur de risque ergonomique ou de charge selon le modèle ergonomique physique est calculée en tant que sortie sur la base de ladite valeur de mesure.

8. Procédé selon la revendication 7, **caractérisé en ce que** le modèle ergonomique physique comprend un modèle d'évaluation HAL et/ou KIM.

9. Procédé selon l'une quelconque des revendications précédentes, **caractérisé en ce que** l'équipement d'assistance de force (110) est agencé spécifiquement pour assister la force de l'épaule, du bras, de la main, de la jambe et/ou du torse de l'utilisateur.

10. Procédé selon l'une quelconque des revendications précédentes, **caractérisé en ce que** le procédé comprend une étape de décision dans laquelle la véritable valeur de risque ergonomique ou de charge calculée et éventuellement également une hypothétique valeur de risque ergonomique ou de charge calculée sont utilisées pour prendre une décision affectant la façon dont, si, pendant combien de temps et le moment où la tâche de travail est réalisée.

11. Procédé selon la revendication 10, **caractérisé en ce que** l'étape de décision comprend une comparaison entre premièrement la véritable valeur de risque ergonomique ou de charge calculée, sous la forme d'une valeur de risque ou de charge instantanée ou une valeur totale de risque ou de charge intégrée, et deuxièmement une valeur de seuil correspondante prédéterminée, et si la véritable valeur de risque ergonomique ou de charge calculée s'avère supérieure à ladite valeur de seuil pour obtenir un signal d'alarme.

**12.** Procédé selon la revendication 11, **caractérisé en ce que** le procédé comprend l'étape consistant à amener l'équipement d'assistance de force (110) à limiter les mouvements de l'utilisateur à la suite de la détection dudit signal d'alarme.

**13.** Procédé selon la revendication 12, **caractérisé en ce que** ladite limitation est obtenue par l'équipement d'assistance de force (110) refusant à l'utilisateur la force d'assistance suffisante pour pouvoir réaliser une tâche de travail prédéterminée nécessitant que l'utilisateur utilise une force dépassant une valeur de seuil de force, ou que l'utilisateur soit alerté concernant le signal d'alarme détecté.

**14.** Système destiné à être utilisé lorsqu'un utilisateur réalise une tâche de travail, dans laquelle l'utilisateur porte et utilise une pièce pouvant être portée activement commandée d'équipement d'assistance de force (110) utilisée pour aider l'utilisateur à réaliser ladite tâche de travail, lequel équipement d'assistance de force (110) comprend un moyen d'exercice de force (112, 113) pour aider l'utilisateur à appliquer une force particulière et/ou pour réaliser des mouvements d'un type particulier, et un moyen de capteur (115) pour détecter une force appliquée par l'utilisateur, dans lequel une force d'assistance exercée par ledit moyen d'exercice de force (112, 113) est commandée par rétroaction sur la base d'une valeur de mesure instantanée provenant du moyen de capteur (115), **caractérisé en ce que** le système comprend un moyen de mesure agencé pour, pendant la réalisation de ladite tâche de travail, mesurer ladite valeur de mesure en utilisant le moyen de capteur (115) destiné à être utilisé dans ladite commande de rétroaction, **en ce que** le système comprend en outre un moyen de calcul agencé pour, en utilisant la valeur de mesure, calculer automatiquement une véritable valeur de risque ergonomique ou de charge pour la tâche de travail réalisée en utilisant ledit équipement d'assistance de force (110), et **en ce que** le moyen de calcul est en outre agencé pour réaliser ledit calcul automatique en utilisant un modèle ergonomique physique déterminé, lequel modèle est spécifique à ladite tâche de travail et/ou à ladite pièce d'équipement d'assistance de force (110), lequel modèle est déterminé sur la base des caractéristiques physiques de la tâche de travail et/ou des caractéristiques physiques du moyen de capteur (115), et lequel modèle comprend en outre une transformation d'une force d'assistance appliquée par ledit équipement d'assistance de force en une force correspondante communiquée par les muscles squelettiques de l'utilisateur.

**15.** Produit de logiciel destiné à être utilisé lorsqu'un utilisateur réalise une tâche de travail, dans laquelle l'utilisateur porte et utilise une pièce pouvant être portée activement commandée d'équipement d'assistance de force (110) utilisée pour aider l'utilisateur à réaliser ladite tâche de travail, lequel équipement d'assistance de force (110) comprend un moyen d'exercice de force (112, 113) pour aider l'utilisateur à appliquer une force particulière et/ou pour réaliser des mouvements d'un type particulier, et un moyen de capteur (115) pour détecter une force appliquée par l'utilisateur, dans lequel une force d'assistance exercée par ledit moyen d'exercice de force (112, 113) est commandée par rétroaction sur la base d'une valeur de mesure instantanée provenant du moyen de capteur (115), **caractérisé en ce que** le produit de logiciel est agencé pour réaliser les étapes consistant à :

a) pendant la réalisation de ladite tâche de travail, réaliser ladite boucle de commande à rétroaction ;
b) également pendant la réalisation de ladite tâche de travail, recevoir ladite valeur de mesure du moyen de capteur (115) destinée à être utilisée dans ladite commande de rétroaction ; et
c) utiliser la valeur de mesure pour calculer automatiquement une véritable valeur de risque ergonomique ou de charge pour la tâche de travail réalisée en utilisant ledit équipement d'assistance de force (110),

et **en ce que** le calcul automatique à l'étape c est réalisé en utilisant un modèle ergonomique physique, lequel modèle est spécifique à ladite tâche de travail et/ou à ladite pièce d'équipement d'assistance de force (110), lequel modèle est déterminé sur la base des caractéristiques physiques de la tâche de travail et/ou des caractéristiques physiques du moyen de capteur (115), et lequel modèle comprend en outre une transformation d'une force d'assistance appliquée par ledit équipement d'assistance de force en une force correspondante communiquée par les muscles squelettiques de l'utilisateur.

# Fig. 1

# Fig. 2

# Fig. 3

| 10 | Start |
|----|-------|
| 11 | Connect force-assisting equipment to server or computer / determine bimechanical model / install software |
| 12 | Initiate work task |
| 13 | Measure force |
| 14 | Communicate measurement value to server or computer |
| 15 | Calculate actual/hypothetical ergonomic risk or load value |
| 16 | Store the actual/hypothetical ergonomic risk or load value |
| 17 | Perform work task analysis and/or user analysis |
| 18 | Take action |
| 19 | End |

**EP 3 801 434 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130226350 A1
- WO 2008027002 A